# EUROPÄISCHE PATENTSCHRIFT

(11) **EP 1 632 562 B1**
(45) Veröffentlichungstag und Bekanntmachung des Hinweises auf die Patenterteilung: **10.01.2007**
(21) Anmeldenummer: 05013493.1
(22) Anmeldetag: 22.06.2005
(51) Int. Cl.: C12M 1/00

(54) **Photobioreaktor**
Photobioreactor
Photobioreacteur

(30) Priorität: 06.09.2004 DE 102004043435
(43) Veröffentlichungstag der Anmeldung: 08.03.2006
(73) Patentinhaber: Infors AG, 4103 Bottmingen (CH)
(72) Erfinder: Hawrylenko, Alexander, 4103 Bottmingen (CH)
(74) Vertreter: Säger, Manfred

(56) Entgegenhaltungen:
- FR-A- 2 564 855
- GB-A- 2 118 572
- US-A1- 2003 073 231

## Beschreibung

Die Erfindung betrifft einen Reaktor gemäss dem Oberbegriff des Hauptanspruchs, nämlich einen Reaktor zur photochemischen Züchtung einer flüssigen Kultur von Klein- und Kleinstlebewesen, vorzugsweise Algen, mit einem Tank, mit einem daran angeschlossenen Rücksowie Vorlauf und mit einer von letzterem in Fliessrichtung gespeisten Vielzahl von zueinander parallelen Rohren, die paarweise an ihren Enden über ein Verbindungsstück miteinander unter Bewirkung eines mäanderförmigen Strömungsverlaufs der flüssigen Kultur durch die Rohre verbunden sind und die vom letzten Rohr in den Rücklauf münden, wobei in der Vielzahl der Rohre die Kultur der Lichtstrahlung einer Lichtquelle für die photochemische Züchtung ausgesetzt ist.

Ein solcher Reaktor ist bekannt (GB 2 118 572 A). Von Nachteil bei diesem bekannten sowie auch anderen Reaktoren ist die Tatsache, dass die gezüchteten und im Kreislauf geführten Kulturen sich letztlich mit der Folge absetzen, dass das für die Züchtung notwendige Licht in einem Masse gedämpft wird, dass die Züchtung mit gutem Wirkungsgrad nicht mehr möglich ist. Aus diesem Grunde muss die Anlage angehalten und die Züchtung der Kultur unter- oder abgebrochen werden, weil die Rohre von ihrem inneren Belag der Kultur befreit werden müssen. Um dies richtig durchzuführen, muss die Anlage im Bereich der Rohrführung jedenfalls zerlegt und im zerlegten Zustand gereinigt werden, was aufwändig ist und zu längeren Unterbrechungen führt, was in hohem Masse unerwünscht ist.

Der Erfindung liegt die Aufgabe zugrunde, einen gattungsgemässen Reaktor gemäss dem Oberbegriff des Hauptanspruchs so auszugestalten, dass eine einfachste Reinigung, ohne die Notwendigkeit zur Zerlegung des Reaktors, zumindest im Bereich der Rohre und der Lichtquelle möglich ist.

Diese Aufgabe wird bei einem gattungsgemässen Reaktor gemäss dem Oberbegriff des Hauptanspruchs erfindungsgemäss durch dessen kennzeichnende Merkmale, also dadurch gelöst, die Lichtquelle als Vielzahl von stabförmigen Lampen ausgebildet ist, dass symmetrisch zu und in jedem Rohr je eine stabförmige Lampe so angeordnet ist, dass deren Achsen parallel zueinander verlaufen, dass das Verbindungsstück auf seiner Vorderseite mit zwei voneinander beabstandeten Öffnungen zur dichten Aufnahme des einen Endes je eines Rohrs versehen ist, dass im Inneren des Verbindungsstücks dessen von den Rohren abgewandte Rückseite mit zwei voneinander beabstandeten Ausnehmungen für je ein Ende einer der Vielzahl von stabförmigen Lampen versehen ist und dass im inneren Bereich der Rückseite des Verbindungsstücks ein Reinigungsschieber angeordnet ist, dessen radiale Innenseite an den Aussenmantel der stabförmigen Lampe form- sowie grössenmässig angepasst ist und der zwecks Reinigung des Aussenmantels der stabförmigen Lampe längs diesem verschiebbar ist.

Durch die Verwendung einer Vielzahl von stabförmigen Lampen kann die Lichteinstrahlung und damit die Geschwindigkeit der Züchtung und Grösse und Menge der gezüchteten Kultur gesteuert werden. Hierbei sind erfindungsgemäss die Vielzahl der stabförmigen Lampen innerhalb der Rohre so angeordnet, dass deren Achsen zueinander parallel sind, vorzugsweise zusammenfallen. Jedes der beiden Enden einer stabförmigen Lampe ist in einem Verbindungsstück gehaltert und über dieses angeschlossen. Dieses Verbindungsstück dient zugleich dazu, die zum Beispiel einen Enden zweier zueinander benachbarter Rohre so miteinander zu verbinden, dass das Innere aller Rohre in Fliessrichtung mäanderförmig miteinander verbunden ist.

Erfindungsgemäss ist nunmehr im inneren Bereich der Rückseite des Verbindungsstücks ein Reinigungsschieber (also innerhalb des Rohrs) angeordnet, dessen Innenseite in jedem Fall an dem Aussenmantel der darin angeordneten stabförmigen Lampe und ggf. dessen Aussenseite an den Innenmantel des Rohrs so angepasst ist, dass wenigstens die Innenseite des Reinigungsschiebers den Belag von dem Aussenmantel der stabförmigen Lampe beim Entlangschieben entfernt; vorzugsweise aber auch zugleich den Innenmantel des Rohrs reinigen kann. So kann mit einer einzigen Bewegung zumindest die Aussenseite der stabförmigen Lampe dadurch gereinigt werden, dass der Reinigungsschieber den durch die Kulturen gebildeten Belag abhebt. Diese Massnahme kann noch durch gleichzeitiges Fluten und Druckspülen des Reaktorkreislaufes sowie andere Reinigungsmassnahmen unterstützt werden.

Mit dieser erfindungsgemässen Ausbildung des Reaktors ist es möglich, dass dieser einfachst und ohne Zerlegung sowie schnell und mit äusserst kurzer Betriebsunterbrechung gereinigt werden kann. Es ist also ein Cleaning In Place (CIP) sowie eine Sterilisierung in situ möglich. Das erfindungsgemässe Prinzip mit den geschilderten Vorteilen ist ohne Vorbild im Stand der Technik.

Gemäss einer Ausführungsform kann durch eine in dem Verbindungsstück ebenfalls vorgesehene, verschlossene sowie zu öffnende Inspektionsöffnung eine Schubstange eingesetzt werden, die in den Reinigungsschieber beispielsweise eingeschraubt wird, sodass dieser in axialer Richtung des Rohrs und der Lampe bis zum anderen Ende geschoben werden kann.

Die bei der vorstehend genannten Ausführungsform notwendige Betriebsunterbrechung muss bei einer bevorzugten Ausführungsform der Erfindung nicht mehr stattfinden, bei der der das Schutzrohr umfassende Reinigungsschieber zumindest ein magnetisches und/oder magnetisierbares angetriebenes Teil aufweist und auf der Innenseite des Schutzrohrs ein die stabförmige Lampe mit Abstand umgebendes, magnetisierbares und/oder magnetisches Antreibteil vorgesehen ist, wobei das angetriebene Teil und das Antreibteil in magnetischer Wirkverbindung stehen, so dass das Antreibteil in dem Schutzrohr über einen nach aussen über das Verbindungsstück geführten Seilzug antreibbar ist.

Weiterhin ist es von Vorteil, wenn das Verbindungsstück mit einer die Kultur tangential an dem Innenmantel des in Fliessrichtung nächstfolgenden Rohrs zur Erzielung einer schraubenförmigen Strömung um die stabförmige Lampe herum führenden Leiteinrichtung versehen ist.

Weitere zweckmässige Ausgestaltungen und Weiterbildungen der Erfindung sind in den Unteransprüchen gekennzeichnet.

Ein Ausführungsbeispiel der Erfindung wird nachfolgend unter Bezugnahme auf die Zeichnung näher erläutert. In dieser zeigt:
- Figur 1: den Reaktor, in Seitenansicht;
- Figur 2: eine Ansicht II-II gemäss Figur 1;

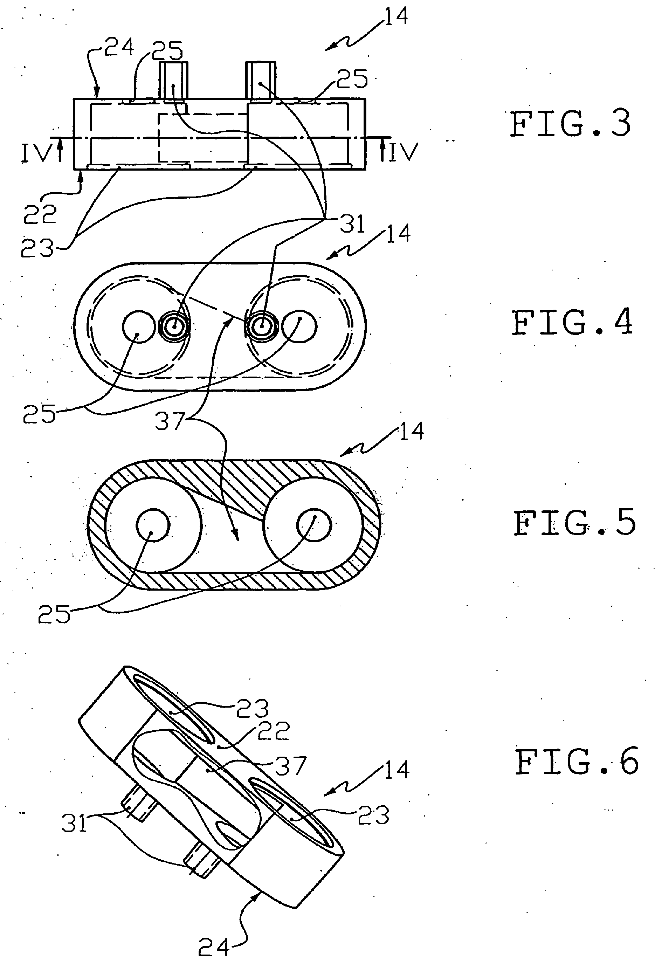

- Figur 3: ein Verbindungsstück, in schematischer Draufsicht;
- Figur 4: einen schematischen Schnitt IV-IV gemäss Figur 3;
- Figur 5: einen weiteren schematischen Schnitt durch das Verbindungsstück;
- Figur 6: das Verbindungsstück in teilweise abgebrochener schematischer perspektivischer Darstellung;
- Figur 7: eine erste Ausführungsform eines Teils des Reaktors mit einem Rohr und einer stabförmigen Lampe, im schematischen, teilweise abgebrochenen Querschnitt;
- Figur 8: den Reaktor gemäss Figur 1, mit schematisch dargestelltem Reinigungsvorgang und in teilweise abgebrochener Darstellung;
- Figur 9: die Einzelheit IX gemäss Figur 8 und Figur 10 eine zweite Ausführungsform des Reaktors.

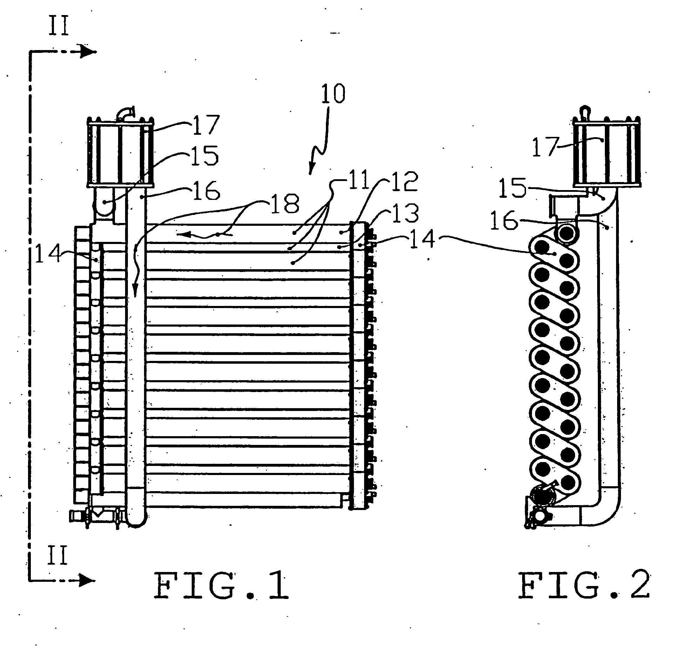

Der in Figur 1 dargestellte Reaktor 10 dient zur photochemischen Züchtung einer flüssigen Kultur von Klein- und Kleinstlebewesen, vorzugsweise Algen. Er weist eine Vielzahl von zueinander parallelen Rohren auf, die jeweils paarweise an benachbarten Enden 12, 13 über ein insgesamt mit 14 bezeichnetes Verbindungsstück miteinander unter Bewirkung eines mäanderförmigen Strömungsverlaufs der flüssigen Kultur durch die Rohre 11 verbunden sind und die von dem letzten Rohr in einen Rücklauf 15 münden, der zu dem Tank 17 führt, von welchem der Vorlauf 16 zu dem ersten Rohr 11 in Fliessrichtung gemäss Richtungspfeil 18 verläuft, wie schematisch in Figur 1 dargestellt. Figur 2 zeigt eine Ansicht II-II des Reaktors.

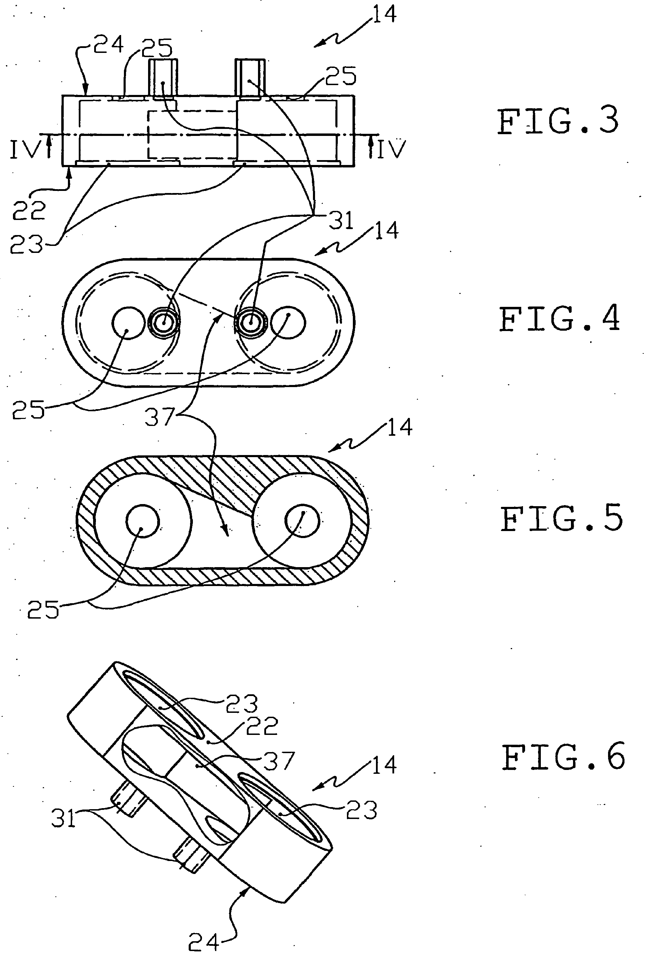

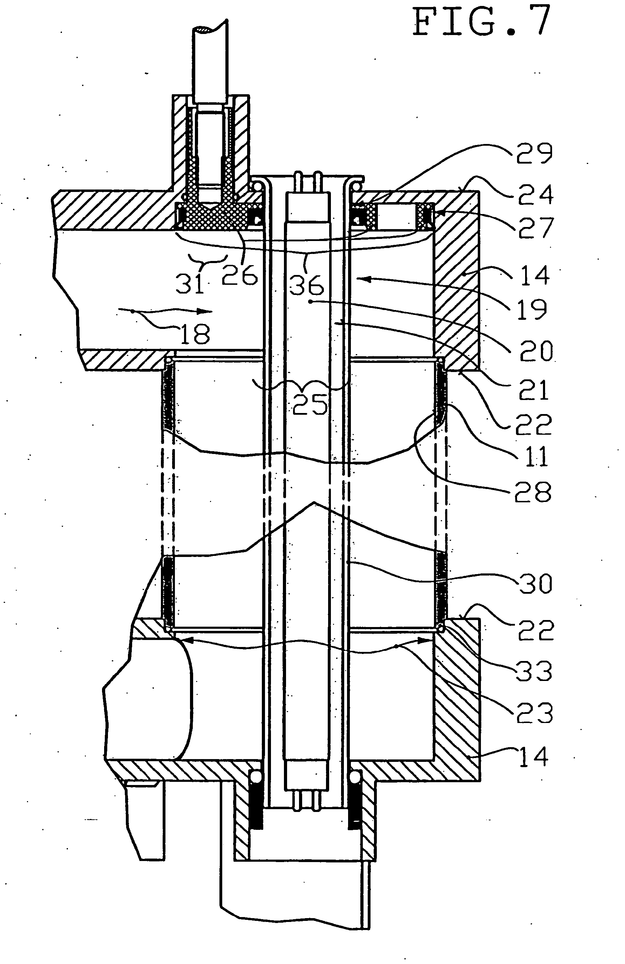

In jedem Rohr 11 ist, wie aus Figur 7 ersichtlich, konzentrisch zu dem kreiszylindrigen Rohr stabförmige Lampen 19 als Lichtquelle so angeordnet, dass deren Achse sowohl parallel zueinander verlaufen als auch mit jener des kreiszylindrigen Rohrs zusammenfallen. Die stabförmige Lampe 19 besteht aus einer eigentlichen elektrischen stabförmigen Lampe 20 und einem diese symmetrisch sowie konzentrisch umgebenden Schutzrohr 21. Das Verbindungsstück 14 ist auf seiner Vorderseite 22 mit zwei voneinander beabstandeten Öffnungen 23 zur dichten Aufnahme des einen Endes je eines Rohrs 11 auf (Figuren 6 und 7) versehen. Ferner ist das Verbindungsstück 14 im Inneren an dessen den Rohren 11 zugewandten Rückseite 24 mit zwei voneinander beabstandeten Ausnehmungen 25 für die stabförmige Lampe 19 versehen.

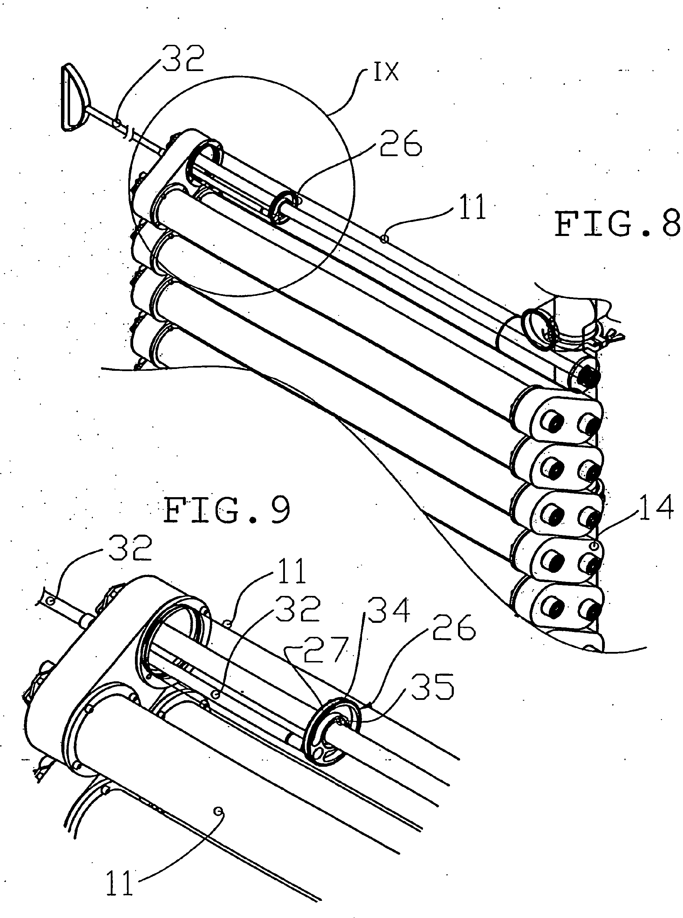

Ferner ist im inneren Bereich der Rückseite 24 des Verbindungsstücks 14 ein insgesamt mit 26 bezeichneter Reinigungsschieber angeordnet, dessen radiale Aussenseite 27 mit dem Innenmantel 28 des Rohrs 11 und dessen Innenseite 29 an den Aussenmantel 30 der stabförmigen Lampe 19 angepasst ist. Ferner ist das Verbindungsstück 14 mit einer dicht schliessbaren Inspektionsöffnung 31 versehen, die mit der Aussenseite (Umgebung) des Verbindungsstücks 14 beim Öffnen, z. B. beim Aufschrauben verbindbar ist. Im geöffneten Zustand kann eine Schubstange 32 (Figur 8 und 9) durch die Inspektionsöffnung hineingesteckt und in den Reinigungsschieber 26 beispielsweise eingeschraubt werden, so dass dieser dann axial in dem Rohr 11 verschoben werden kann, wobei in jedem Falle der Aussenmantel 21 der stabförmigen Lampe 19, aber auch der Innenmantel 28 des Rohrs 11 von allfälligen Belägen gereinigt werden kann.

Die Öffnung 23 an der Vorderseite 22 des Verbindungsstücks 14 ist mit einem Absatz 33 versehen, gegen den sich das Ende des betreffenden Rohrs 11 abstützen kann. Die Ausnehmung 25 auf der Rückseite 24 im Inneren des Verbindungsstücks 14 ist hierbei konzentrisch zu der Öffnung 23 auf der Vorderseite 22 des Verbindungsstücks 14 angeordnet, wobei die Vorderseite 22 und die Rückseite 24 parallel zueinander verlaufen. Auf der Rückseite 24 des Verbindungsstücks 14 sind die zwei Inspektionsöffnungen 31 neben der Ausnehmung 25 so vorgesehen, dass sich jede Inspektionsöffnung 31 mit der zugehörigen Öffnung 23 auf der Vorderseite 22 des Verbindungsstücks 14 zumindest teilweise überlappt. Durch diese Geometrie ist sichergestellt, dass der Reinigungsschieber 26, der im wesentlichen aus zwei zueinander konzentrischen, starr miteinander verbundenen Ringen 34, 35, besteht, in das Innere des Rohrs geschoben werden kann, um den eingangs erwähnten Reinigungseffekt zu erzielen. Zweckmässigerweise ist der Reinigungsschieber 26 deshalb mit einer Gewindebohrung zur Aufnahme des Gewindes an der Vorderseite der Schubstange 32 versehen.

Um in Betrieb die Strömung in dem Verbindungsstück 14 nicht zu beeinflussen, ist in dem Inneren Bereich der Rückseite 24 des Verbindungsstücks 14 um die Ausnehmung 25 herum eine Vertiefung 36 für den Reinigungsschieber 26 vorgesehen, wie das in Figur 7 schematisch dargestellt ist. Aus Figur 7 ist ersichtlich, dass es für eines der Rohre ausreicht, wenn das an seinem einen Ende vorgesehene Verbindungsstück 14 mit einer Inspektionsöffnung 31 versehen ist, wohingegen das am anderen Ende des Rohrs 11 befindliche Verbindungsstück dann eine solche Inspektionsöffnung beziehungsweise einen Reinigungsschieber nicht benötigt.

Mit ganz besonderem Vorteil wird bei der Erfindung nicht nur das einfache Entfernen zumindest des Belags auf dem Aussenmantel der stabförmigen Lampe 19 ermöglicht. Durch eine Leiteinrichtung des Verbindungsstücks 14 kann die Kultur tangential an den Innenmantel 28 des in Fliessrichtung 18 nächstfolgenden Rohrs 11 mit einer schraubenförmig verlaufenden Strömung um die stabförmige Lampe 19 angeströmt werden. Hierdurch wird die Möglichkeit der Einstrahlung auf die flüssige Kultur reduziert und damit der Wirkungsgrad des Reaktors verkleinert.

Die Leiteinrichtung 37 des Verbindungsstücks 14 ist als Leitfläche ausgebildet, die sich tangential von der einen, an der Vorderseite 22 befindlichen Öffnung 23 zur Aufnahme des einen Endes des einen Rohrs 11 in Fliessrichtung 18 zur nächstfolgenden anderen Öffnung für das Ende des anderen Rohrs im Sinne einer inneren Tangentenfläche, die vorzugsweise eine innere Tangenteneben sein kann, erstreckt.

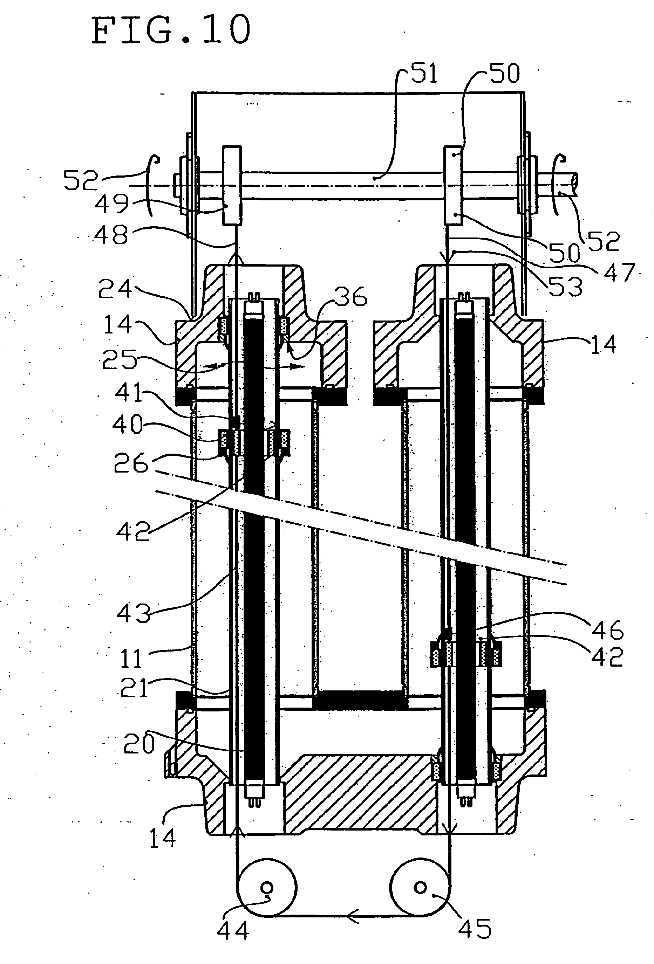

Ferner ist in Figur 10 eine zweite, bevorzugte Ausführungsform der Erfindung im schematischen Querschnitt dargestellt. Hierbei bezeichnen die gleichen Bezugszeichen, die schon im Zusammenhang mit den vorherigen Figuren beschriebenen Einzelheiten. Ferner weist der das Schutzrohr 21 umgreifende Reinigungsschieber 26 zumindest ein magnetisches und/oder magnetisierbares angetriebenes Teil 40 auf. Auf der Innenseite 41 des Schutzrohrs 21 ist ferner ein die stabförmige Lampe 20 umgebendes, magnetisierbares und/oder magnetisches Antreibteil 42 vorgesehen, wobei dieses und das angetriebene Teil in magnetischer Wirkverbindung stehen. Somit kann das angetriebene Teil 40 durch das Schutzrohr 21 hindurch gewissermassen von innen her längs dessen Aussenseite zum Abheben des durch die Kultur gebildeten Belages geführt werden. Hierzu wird das Antreibteil 42 durch eine über das Verbindungsstück 14 innerhalb des Schutzrohrs 21 nach aussen geführten Seilzug 43 antreibbar ausgebildet. Zweckmässigerweise ist nur ein einziger Seilzug 43 für die beiden Schutzrohre 21 vorgesehen, die mit dem selben Verbindungsstück 14 (in Figur 10, oben) miteinander verbunden sind. Hierbei wird derselbe Seilzug 43 im Bereich des gemeinsamen Verbindungsstücks 14, jedoch ausserhalb des Reaktors mittels zweier Umlenkrollen 44, 45 umgelenkt. Jedes Antreibteil 42 ist mit diesem gemeinsamen Seilzug 43 vorzugsweise über eine Feder 46 verbunden. An dem bezüglich des gemeinsamen Verbindungsstücks 14 entgegengesetzten Ende 47, 48 des gemeinsamen Seilzugs 43 sind auf je eine Aufwickeltrommel 49 beziehungsweise 15 aufgewickelt. Die beiden Auf- beziehungsweise Abwickeltrommeln 49, 50 sitzen auf derselben Antriebswelle 51, die gemäss Richtungspfeil 52 im Betrieb gedreht wird, sodass die eine Trommel 50 abgewickelt und die andere Trommel 49, wie aus dem Richtungspfeil 53 ersichtlich, aufgewickelt wird.

Der Reinigungsschieber 26 mit dessem magnetischen angetriebenen Teil 40 weist in seiner einen Endlage (wie aus Figur 10) im linken Schutzrohr 21, oben und im rechten Schutzrohr 21, unten gezeigt - eine quasi Parkposition innerhalb des für das - nicht gemeinsamen - Verbindungsstücks 14 auf, indem in dem inneren Bereich der Rückseite 24 des Verbindungsstücks um die Ausnehmung 25 für das Schutzrohr 21 herum eine Vertiefung 36 für den Reinigungsschieber 26 vorgesehen ist.

Bei dieser zweiten Ausführungsform des erfindungsgemässen Reaktors ist es möglich, nicht nur ein CIP sowie eine Sterilisierung in situ vorzusehen, sondern darüber hinaus auch noch während des Betriebs des Reaktors, in dem beispielsweise nach fest vorgegebenen Betätigungsintervallen, zum Beispiel alle 30 oder 45 Minuten der Reinigungsschieber für eine Hin- und Herbewegung aus seiner quasi Parkposition einmal hin und in diese wieder zurückbewegt wird. Die Anlage braucht daher zur Reinigung weder angehalten, noch zerlegt werden zu müssen.

## Patentansprüche

1. Reaktor (10) zur photochemischen Züchtung einer flüssigen Kultur von Klein- und Kleinstlebewesen, vorzugsweise Algen, mit einem Tank (17), mit einem daran angeschlossenen Rück- sowie Vorlauf (15 bzw. 16) und mit einer von letzterem in Fliessrichtung (18) gespeisten Vielzahl von zueinander parallelen Rohren (11), die paarweise an ihren Enden (12,13) über ein Verbindungsstück (14) miteinander unter Bewirkung eines mäanderförmigen Strömungsverlaufs der flüssigen Kultur durch die Rohre (11) verbunden sind und die vom letzten Rohr in den Rücklauf (15) münden, wobei in der Vielzahl der Rohre (11) die Kultur der Lichtstrahlung einer Lichtquelle für die photochemische Züchtung ausgesetzt ist,
**dadurch gekennzeichnet, dass** die Lichtquelle als Vielzahl von einen Aussenmantel (30) aufweisenden stabförmigen Lampen (19) ausgebildet ist, dass symmetrisch zu und in jedem Rohr (11) je eine stabförmige Lampe (19) so angeordnet ist, dass deren Achsen parallel zueinander verlaufen, dass das Verbindungsstück (14) auf seiner Vorderseite (22) mit zwei voneinander beabstandeten Öffnungen (23) zur dichten Aufnahme des einen Endes je eines Rohrs (11) versehen ist, dass im Inneren des Verbindungsstücks (14) dessen von den Rohren (11) abgewandte Rückseite (24) mit zwei voneinander beabstandeten Ausnehmungen (25) für je ein Ende einer der Vielzahl von stabförmigen Lampen (19) versehen ist und dass im inneren Bereich der Rückseite des Verbindungsstücks (14) ein Reinigungsschieber (26) angeordnet ist, dessen radiale Innenseite (29) an den Aussenmantel (30) der stabförmigen Lampe (19) form- sowie grössenmässig angepasst ist und der zwecks Reinigung des Aussenmantels (30) der stabförmigen Lampe (19) längs diesem verschiebbar ist.

2. Reaktor nach Anspruch 1, **dadurch gekennzeichnet, dass** die stabförmige Lampe (19) aus einer eigentlichen elektrischen, stabförmigen Lampe (20) sowie einem diese symmetrisch umgebenden, den Aussenmantel (30) aufweisenden äusseren Schutzrohr (21) besteht und dass jede stabförmige Lampe (19) konzentrisch in dem Rohr (11) angeordnet ist.

3. Reaktor nach Anspruch 1 oder 2, **dadurch gekennzeichnet, dass** der Reinigungsschieber (26) mittels einer durch eine den inneren Bereich der Rückseite (24) des Verbindungsstücks (14) mit dessen Ausssenseite verbindenden, dicht schliessbaren Inspektionsöffnung (31) steckbaren Schubstange (32) zwecks Reinigung des Aussenmantels (30) der stabförmigen Lampe (19) verschiebbar ist.

4. Reaktor nach Anspruch 1, 2 oder 3, **dadurch gekennzeichnet, dass** die radiale Aussenseite (27) des Reinigungsschiebers (26) an den Innenmantel (28) des Rohrs (11) angepasst ist und dass zwecks gleichzeitiger Reinigung dieses Innenmantels (28) des Rohrs (11) der Reinigungsschieber (26) mittels der durch die den inneren Bereich der Rückseite (24) des Verbindungsstücks (14) mit dessen Ausssenseite verbindenden, dicht schliessbaren Inspektionsöffnung (31) steckbaren Schubstange (32) verschiebbar ausgebildet ist.

5. Reaktor nach einem der Ansprüche 1 bis 4, **dadurch gekennzeichnet, dass** die Öffnung (23) in dem Verbindungsstück (14) mit einem Absatz (33) versehen ist, gegen den sich das Ende (12,13) des Rohrs (11) abstützt.

6. Reaktor nach Anspruch einem der Ansprüche 1 bis 5, **dadurch gekennzeichnet, dass** die Ausnehmung (25) auf der Rückseite (24) konzentrisch zu der zugehörigen Öffnung (23) auf der Vorderseite (22) des Verbindungsstücks (14) angeordnet ist und die Rückseite (24) sowie die Vorderseite (22) parallel zueinander verlaufen.

7. Reaktor nach einem der Ansprüche 1 bis 6, **dadurch gekennzeichnet, dass** auf der Rückseite (24) des Verbindungsstücks (14) zwei Inspektionsöffnungen (31) neben der Ausnehmung (25) so vorgesehen sind, dass sich jede Inspektionsöffnung (31) mit der zugehörigen Öffnung (23) auf der Vorderseite (22) des Verbindungsstücks (14) zumindest teilweise überlappt.

8. Reaktor nach einem der Ansprüche 1 bis 7, **dadurch gekennzeichnet, dass** der Reinigungsschieber (26) im wesentlichen aus zwei zueinander konzentrischen, starr miteinander verbunden Ringen (34, 35), nämlich einen seine radiale Aussenseite (27) bildenden Ring (34) sowie einem seine radiale Innenseite (29) bildenden Ring (35) besteht.

9. Reaktor nach einem der Ansprüche 1 bis 8, **dadurch gekennzeichnet, dass** der Reinigungsschieber (26) eine Gewindebohrung zur Aufnahme des Aussengewindes an der Vorderseite der Schubstange (32) aufweist.

10. Reaktor nach einem der Ansprüche 1 bis 9, **dadurch gekennzeichnet, dass** in dem inneren Bereich der Rückseite (24) des Verbindungsstücks (14) um die Ausnehmung (25) herum eine Vertiefung (36) für den Reinigungsschieber (26) vorgesehen ist.

11. Reaktor nach Anspruch 1 oder 2, **dadurch gekennzeichnet, dass** der das Schutzrohr (21) umgreifende Reinigungsschieber (26) zumindest ein magnetisches und/oder magnetisierbares angetriebenes Teil (40) aufweist, dass auf der Innenseite des Schutzrohrs (21) ein die stabförmige Lampe (20) mit Abstand umgebendes, magnetisierbares und/oder magnetisches Antreibteil (42) vorgesehen ist und dass dieses und das angetriebene Teil in magnetischer Wirkverbindung stehen.

12. Reaktor nach Anspruch 11, **dadurch gekennzeichnet, dass** das Antreibteil (42) in dem Schutzrohr (21) über einen über das Verbindungsstück (14) nach aussen geführten Seilzug (43) antreibbar ist.

13. Reaktor nach Anspruch 12, **dadurch gekennzeichnet, dass** die in den beiden mit demselben Verbindungsstück (14) verbundenen Schutzrohre (21) vorgesehenen Antreibteile (42) über denselben Seilzug (43) antreibbar sind.

14. Reaktor nach Anspruch 13, **dadurch gekennzeichnet, dass** derselbe Seilzug (43) im Bereich des gemeinsamen Verbindungsstücks (14) mittels zweier ausserhalb des Reaktors befindlicher Umlenkrollen (44, 45) umgelenkt ist.

15. Reaktor nach Anspruch 14, **dadurch gekennzeichnet, dass** im Bereich der bezüglich des gemeinsamen Verbindungsstücks (14) entgegengesetzen Enden beider Schutzrohre (21) jedes Ende des gemeinsamen Seilzugs (43) auf je eine Auf- bzw. Abwickeltrommel aufgewickelt ist.

16. Reaktor nach Anspruch 15, **dadurch gekennzeichnet, dass** die beiden Auf- bzw. Abwickeltrommeln auf derselben Antriebswelle sitzen und im Betrieb die eine auf- während die andere abgewickelt wird.

17. Reaktor nach einem der Ansprüche 11 bis 16, **dadurch gekennzeichnet, dass** in dem inneren Bereich der Rückseite (24) des Verbindungsstücks (14) um die Ausnehmung (25) für das Schutzrohr (21) herum eine Vertiefung (36) für den Reinigungsschieber (26) vorgesehen ist.

18. Reaktor nach einem der Ansprüche 1 bis 17, **dadurch gekennzeichnet, dass** das Verbindungsstück (14) mit einer die Kultur tangential an den Innenmantel (28) des in Fliessrichtung (18) nächstfolgenden Rohrs (11) heranund zur Erzielung einer schraubenförmigen Strömung um die stabförmige Lampe (19) herum führenden Leiteinrichtung (37) versehen ist.

## Claims

1. Reactor (10) for the photo-chemical cultivation of a liquid culture of organisms and micro-organisms, preferably algae, comprising a tank (17) with forward and return flow (15 and respectively 16) connected to the latter and comprising a plurality of mutually-parallel tubes (11) supplied from the latter in the direction of flow (18), which are connected together in pairs at their ends (12, 13) via a connecting piece (14) thereby achieving a meandering flow-characteristic of the liquid culture through the tubes (11, and which open from the last tube into the return flow (15), wherein the culture is exposed within the plurality of tubes (11) to an irradiation with light from a light source in order to achieve the photo-chemical cultivation,
**characterised in that**
the light source is formed as a plurality of rod-shaped lamps (19) providing an outer casing (30); that one rod-shaped lamp (19) is arranged symmetrically within each tube (11) in such a manner that their axes extend parallel to one another;
that the connecting piece (14) is provided with two mutually-spaced openings (23) on its front side (22) in order to accommodate one end of each tube (11) in a sealed manner;
that in the interior of the connecting pieces (14), their rear side (24) facing away from the tubes (11) is provided with two mutually-spaced recesses (25) in each case for one end of one of the plurality of rod-shaped lamps (19);
and that a cleaning slider (26), of which the radially-internal side (29) is adapted in shape and size to the outer casing (30) of the rod-shaped lamp (19), and which can be displaced along the latter for the purpose of cleaning the outer casing (30) of the rod-shaped lamp (19), is arranged in the interior region of the rear side of the connecting piece (14).

2. Reactor according to claim 1,
**characterised in that**
the rod-shaped lamp (19) consists of an actual electrical, rod-shaped lamp (20) and an outer protective tube (21) surrounding the latter symmetrically and providing the outer casing (30); and that each rod-shaped lamp (19) is arranged in a concentric manner within the tube (11).

3. Reactor according to claim 1 or 2,
**characterised in that**
the cleaning slider (26) can be displaced, for the purpose of cleaning the outer casing (30) of the rod-shaped lamp (19), by means of a push rod (32), which can be inserted through a tightly-sealable inspection opening (31), which connects the interior region of the rear side (24) of the connecting piece (14) with its exterior side.

4. Reactor according to claim 1, 2 or 3,
**characterised in that**
the radially-external side (27) of the cleaning slider (26) is adapted to the inner casing (28) of the tube (11); and that, for the purpose of a simultaneous cleaning of this inner casing (28) of the tube (11), the cleaning slider (26) is designed to be displaceable by means of the push rod (32), which can be inserted through the tightly-sealable inspection opening (31) connecting the interior region of the rear side (24) of the connecting piece (14) to its exterior side.

5. Reactor according to any one of claims 1 to 4,
**characterised in that**
the opening (23) in the connecting piece (14) is provided with a shoulder (33), against which the end (12, 13) of the tube (11) is supported.

6. Reactor according to any one of claims 1 to 5,
**characterised in that**
the recess (25) at the rear side (24) is arranged concentrically to the associated opening (23) at the front side (22) of the connecting piece (14), and the rear side (24) and the front side (22) extend parallel to one another.

7. Reactor according to any one of claims 1 to 6,
**characterised in that**
two inspection openings (31) are provided in the rear side (24) of the connecting piece (14) adjacent to the recess (25) in such a manner that each inspection opening (31) overlaps at least partially with the associated opening (23) in the front side (22) of the connecting piece (14).

8. Reactor according to any one of claims 1 to 7
**characterised in that**
the cleaning slider (26) essentially consists of two mutually-concentric, rigidly-interconnected rings (34, 35), wherein one ring (34) forms its radially-external side (27) and one ring (35) forms its radially-internal side (29).

9. Reactor according to any one of claims 1 to 8,
**characterised in that**
the cleaning slider (26) provides a threaded borehole to accommodate the external thread at the front end of the push rod (32).

10. Reactor according to any one of claims 1 to 9,
**characterised in that**
an indentation (36) is provided for the cleaning slider (26) around the recess (25) in the interior region of the rear side (24) of the connecting piece (14).

11. Reactor according to claim 1 or 2,
**characterised in that**
the cleaning slider (26) engaging around the protective tube (21) has at least one magnetic and/or magnetisable, driven component (40); that a magnetisable and/or magnetic drive component (42) is provided on the internal side of the protective tube (21), surrounding the rod-shaped lamp (20) at a spacing distance; and that the drive component and the driven component are connected by an active magnetic connection.

12. Reactor according to claim 11,
**characterised in that**
the drive component (42) can be driven within the protective tube (21) via a line-pull (43) guided through the connecting piece (14) to the outside.

13. Reactor according to claim 12,
**characterised in that**
the drive components (42) provided in two protective tubes (21) connected by the same connecting piece (14) can be driven via the same line-pull (43).

14. Reactor according to claim 13,
**characterised in that**
the same line-pull (43) is deflected in the region of the common connecting piece (14) by means of two deflection pulleys (44, 45) disposed outside the reactor.

15. Reactor according to claim 14,
**characterised in that**
each end of the common line-pull (43) is wound from a supply drum and respectively onto a take-up drum in the region of the ends of both protective tubes (21) disposed opposite to the common connecting piece (14).

16. Reactor according to claim 15,
**characterised in that**
the two supply and take-up drums are mounted on the same drive shaft, and during operation, one drum supplies while the other drum takes up.

17. Reactor according to any one of claims 11 to 16,
**characterised in that**
an indentation (36) is provided for the cleaning slider (26) around the recess (25) for the protective tube (21) in the interior region of the rear side (24) of the connecting piece (14).

18. Reactor according to any one of claims 1 to 17,
**characterised in that**
the connecting piece (14) is provided with a guide device (37) guiding the culture in the direction of flow (18) tangentially towards the inner casing (28) of the respective next tube (11) and around the rod-shaped lamp (19) in order to achieve a helical flow.

## Revendications

1. Réacteur (10) pour la multiplication photochimique d'une culture liquide de mini-organismes et de micro-organismes, de préférence des algues, comportant une cuve (17) à laquelle sont raccordés un retour ainsi qu'un départ (15 ou 16), et comportant une pluralité de tubes (11) parallèles les uns aux autres alimentés par ces derniers dans le sens de l'écoulement (18), qui sont raccordés les uns aux autres par paires à leurs extrémités (12, 13) par l'intermédiaire d'un raccord (14) en créant une trajectoire d'écoulement en méandres de la culture liquide à travers les tubes (11), et qui débouchent par le dernier tube dans le retour (15), moyennant quoi la culture est exposée dans la pluralité de tubes (11) au rayonnement lumineux d'une source lumineuse pour la multiplication photochimique,
**caractérisé en ce que** la source lumineuse est réalisée sous la forme d'une pluralité de lampes en forme de barres (19) présentant une enveloppe extérieure (30), **en ce que**, dans chaque tube (11) et symétriquement par rapport à celui-ci est agencée une lampe en forme de barre (19) de telle manière que leurs axes sont parallèles l'un à l'autre, **en ce que** le raccord (14) est muni, sur sa face avant (22), de deux ouvertures (23) espacées l'une de l'autre pour loger hermétiquement une des extrémités de chacun des tubes (11), **en ce que**, à l'intérieur du raccord (14), dont la face arrière (24) opposée aux tubes (11) est munie de deux évidements (25) espacés l'un de l'autre destinés chacun à une extrémité d'une de la pluralité de lampes en forme de barre (19), et **en ce que**, dans la zone intérieure de la face arrière du raccord (14), est agencé un élément coulissant de nettoyage (26) dont la face intérieure radiale (29) est adaptés à l'enveloppe extérieure (30) de la lampe en forme de barre (19) quant à la forme ainsi qu'aux dimensions, et qui peut coulisser le long de l'enveloppe extérieure (30) de la lampe en forme de barre (19) à des fins de nettoyage de cette dernière.

2. Réacteur selon la revendication 1, **caractérisé en ce que** la lampe en forme de barre (19) est constituée d'une lampe électrique en forme de barre proprement dite (20), ainsi que d'un tube de protection extérieur (21) l'entourant symétriquement et présentant l'enveloppe extérieure (30), et **en ce que** chaque lampe en forme de barre (19) est agencée concentriquement dans le tube (11).

3. Réacteur selon la revendication 1 ou 2, **caractérisé en ce que** l'élément coulissant de nettoyage (26) peut coulisser, à des fins de nettoyage de l'enveloppe extérieure (30) de la lampe en forme de barre (19), au moyen d'une tige de poussée (32) pouvant être introduite par une ouverture de visite (31) pouvant être fermée hermétiquement, reliant la zone intérieure de la face arrière (24) du raccord (14) à sa face extérieure.

4. Réacteur selon la revendication 1, 2 ou 3, **caractérisé en ce que** la face extérieure radiale (27) de l'élément coulissant de nettoyage (26) est adaptée à l'enveloppe intérieure (28) du tube (11), et **en ce qu'**à des fins de nettoyage simultané de cette enveloppe intérieure (28) du tube (11), l'élément coulissant de nettoyage (26) est conçu pour pouvoir coulisser au moyen de la tige de poussée (32) pouvant être introduite par l'ouverture de visite (31) pouvant être fermée hermétiquement, reliant la zone intérieure de la face arrière (24) du raccord (14) à sa face extérieure.

5. Réacteur selon une des revendications 1 à 4, **caractérisé en ce que** l'ouverture (23) prévue dans le raccord (14) est munie d'un épaulement (33) contre lequel prend appui l'extrémité (12, 13) du tube (11).

6. Réacteur selon une des revendications 1 à 5, **caractérisé en ce que** l'évidement (25) est agencé sur la face arrière (24) concentriquement par rapport à l'ouverture correspondante (23) prévue sur la face avant (22) du raccord (14), et **en ce que** la face arrière (24) ainsi que la face avant (22) sont parallèles l'une à l'autre.

7. Réacteur selon une des revendications 1 à 6, **caractérisé en ce que** sur la face arrière (24) du raccord (14) sont prévues deux ouvertures de visite (31) à côté de l'évidement (25) de telle manière que chaque ouverture de visite (31) chevauche au moins partiellement l'ouverture correspondante (23) prévue sur la face avant (22) du raccord (14).

8. Réacteur selon une des revendications 1 à 7, **caractérisé en ce que** l'élément coulissant de nettoyage (26) est constitué principalement de deux bagues (34, 35) concentriques l'une par rapport à l'autre, reliées rigidement l'une à l'autre, à savoir une bague (34) formant sa face extérieure radiale (27) ainsi qu'une bague (35) formant sa face intérieure radiale (29).

9. Réacteur selon une des revendications 1 à 8, **caractérisé en ce que** l'élément coulissant de nettoyage (26) présente un alésage fileté destiné à recevoir le filetage extérieur prévu sur la face avant de la tige de poussée (32).

10. Réacteur selon une des revendications 1 à 9, **caractérisé en ce que** dans la zone intérieure de la face arrière (24) du raccord (14) est prévu autour de l'évidement (25) un renfoncement (36) pour l'élément coulissant de nettoyage (26).

11. Réacteur selon la revendication 1 ou 2, **caractérisé en ce que** l'élément coulissant de nettoyage (26) entourant le tube de protection (21) présente au moins un élément mené magnétique et/ou magnétisable (40), **en ce que** sur la face intérieure du tube de protection (21) est prévu un élément menant magnétisable et/ou magnétique (42) entourant à distance la lampe en forme de barre (20), et **en ce que** ce dernier et l'élément mené sont en liaison active magnétique.

12. Réacteur selon la revendication 11, **caractérisé en ce que** l'élément menant (42) peut être entraîné dans le tube de protection (21) par l'intermédiaire d'un câble de commande (43) guidé vers l'extérieur par l'intermédiaire du raccord (14).

13. Réacteur selon la revendication 12, **caractérisé en ce que** les éléments menants (42) prévus dans les deux tubes de protection (21) raccordés par le même raccord (14) peuvent être entraînés par l'intermédiaire du même câble de commande (43).

14. Réacteur selon la revendication 13, **caractérisé en ce que** le même câble de commande (43) est renvoyé dans la zone du raccord commun (14) au moyen de deux poulies de renvoi (44, 45) se trouvant à l'extérieur du réacteur.

15. Réacteur selon la revendication 14, **caractérisé en ce que**, dans la zone des extrémités des deux tubes de protection (21) opposées par rapport au raccord (14) commun, chaque extrémité du câble commun (43) est enroulée respectivement sur un tambour enrouleur ou dérouleur.

16. Réacteur selon la revendication 15, **caractérisé en ce que** les deux tambours enrouleurs ou dérouleurs se trouvent sur le même arbre d'entraînement, et **en ce que** pendant le fonctionnement, l'un assure l'enroulement pendant que l'autre assure le déroulement.

17. Réacteur selon une des revendications 11 à 16, **caractérisé en ce que** dans la zone intérieure de la face arrière (24) du raccord (14) est prévu autour de l'évidement (25) destiné au tube de protection (21) un renfoncement (36) pour l'élément coulissant de nettoyage (26).

18. Réacteur selon une des revendications 1 à 17, **caractérisé en ce que** le raccord (14) est muni d'un dispositif de guidage (37) guidant la culture tangentiellement contre l'enveloppe intérieure (28) du tube (11) suivant vu dans le sens de l'écoulement (18), pour l'obtention d'un flux hélicoïdal autour de la lampe en forme de barre (19).
